# EUROPEAN PATENT APPLICATION

(11) **EP 4 292 593 A1**
(43) Date of publication of application: **20.12.2023**
(21) Application number: 22752864.3
(22) Date of filing: 14.02.2022
(51) Int. Cl.: A61K 31/352, A23L 33/10, A61P 37/02, A61P 43/00

(54) **PHARMACEUTICAL COMPOSITION FOR PREVENTING CYTOKINE STORM**

(30) Priority: 15.02.2021 JP 2021021955
(71) Applicant: Kinki University, Higashi-Osaka-shi Osaka 577-8502 (JP)
(72) Inventor: NISHIDA Shozo, Higashiosaka City, Osaka 5778502 (JP); TSUBAKI Masanobu, Higashiosaka City, Osaka 5778502 (JP); TAKEDA Tomoya, Higashiosaka City, Osaka 5778502 (JP); TANABE Genzoh, Higashiosaka City, Osaka 5778502 (JP); TAKASHIMA Katsuki, Higashiosaka City, Osaka 5778502 (JP); MORIKAWA Toshio, Higashiosaka City, Osaka 5778502 (JP)
(74) Representative: Grünecker Patent- und Rechtsanwälte PartG mbB
(86) International application number: PCT/JP2022/005709
(87) International publication number: WO 2022/173042

(57) **Abstract**

[Problem]

COVID-19 caused by coronavirus SARS-CoV-2, cytokine release syndrome, cytokine release syndrome with CAR (chimeric antigen receptor)-T therapy, sepsis, systemic inflammatory response syndrome, hemophagocytic syndrome, macrophage activation syndrome, influenza, GVHD, systemic vasculitis, Kawasaki disease, Takayasu arteritis, and other diseases, if severe, can cause acute respiratory distress syndrome, disseminated intravascular coagulation syndrome, acute circulatory insufficiency, multi-organ failure, and other symptoms which are one of the causes of death.

[Solution to Problem]

Compounds represented by formulas (1) to (4) are effective when taken orally and can be expressed in smaller amounts than mangiferin.

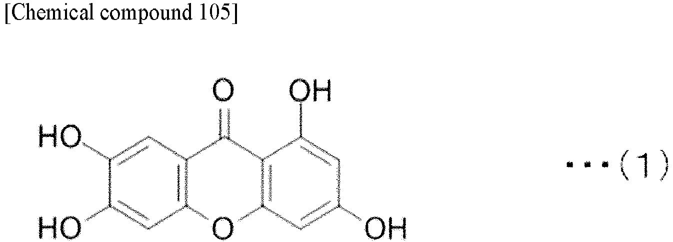

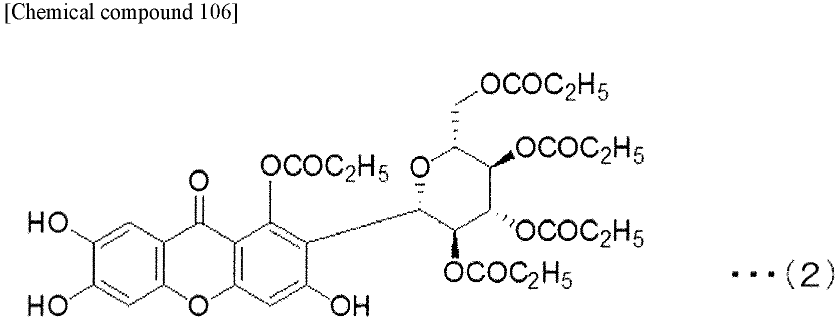

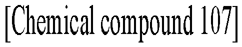

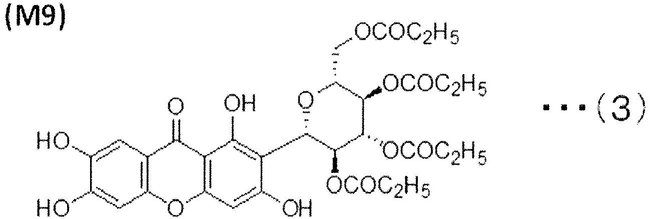

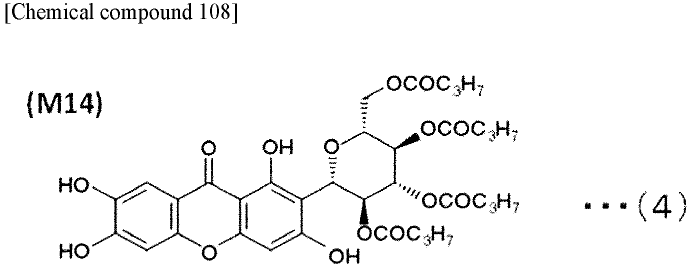

## Description

### [Technical Field]

The present invention relates to compositions, pharmaceutical compositions, processed foods, and agents for the prevention, treatment, and alleviation of cytokine storm, which are a cause of death such as COVID-19, cytokine release syndrome, chimeric antigen receptor (CAR)-T therapy-induced cytokine release syndrome, sepsis, systemic inflammatory response syndrome, hemophagocytic syndrome, macrophage activation syndrome, influenza, graft-versus-host disease (GVHD), systemic vasculitis, Kawasaki disease, Takayasu arteritis, and others.

### [Background Art]

Coronavirus disease 2019 (COVID-19) is a disease caused by severe acute respiratory syndrome coronavirus 2 (SARS-CoV-2). Critically ill patients with this disease develop acute respiratory distress syndrome and acute lung injury.

Inflammatory cytokines such as tumor necrosis factor α (TNFα) and interleukin 6 (IL-6) are important factors in causing these symptoms.

Inflammatory cytokines such as TNFα and IL-6 are secreted by macrophages, B cells, and monocytes, and are released in large amounts by diseases such as COVID-19, cytokine release syndrome, cytokine release syndrome due to CAR-T therapy, sepsis, systemic inflammatory response syndrome, hemophagocytic syndrome, macrophage activation syndrome, influenza, GVHD, systemic vasculitis, Kawasaki disease, Takayasu arteritis, and others.

This massive release of cytokines induces an excessive inflammatory response that causes fatal injuries to various organs. This condition is called cytokine storm and is a factor in acute respiratory distress syndrome, disseminated intravascular coagulation syndrome, acute circulatory failure, and multiorgan failure, and is one of the causes of death.

NIK is a serine/threonine kinase that plays a role in both canonical and non-canonical NF-κB pathways, but is essential in the non-canonical NF-κB signaling pathway, phosphorylating IKKα to partially degrade NF-κB p100 and release NF-κB p52. NF-κB p52 forms heterodimers with RelB, which translocate into the nucleus and express genes for inflammatory cytokines such as TNFα and IL-6. In addition, the canonical NF-κB pathway activates the IKKα, IKKβ, and IKKγ complexes to form a heterodimer of NF-xBp65 and NF-xBp50. This heterodimer translocates into the nucleus to regulate gene expression of inflammatory cytokines such as TNFα and IL-6.

Lipopolysaccharide (LPS), which causes sepsis, hemophagocytic syndrome, and macrophage activation syndrome, acts on macrophages, B cells, monocytes, and others to activate NIK and others. NIK and others activate the canonical and non-canonical NF-κB pathway, which in turn promotes the production of inflammatory cytokines such as TNFα and IL-6 in B cells and macrophages.

Thus, compounds that inhibit canonical and non-canonical NF-κB pathway activation by NIK via LPS or other means and suppress the production of inflammatory cytokines may suppress acute respiratory distress syndrome, disseminated intravascular coagulation syndrome, acute circulation failure, multi organ failure, and other symptoms, which caused by COVID-19, cytokine release syndrome, cytokine release syndrome due to CAR-T therapy, sepsis, systemic inflammatory response syndrome, hemophagocytic syndrome, macrophage activation syndrome, influenza, GVHD, systemic vasculitis, Kawasaki disease, Takayasu arteritis, and others. Suppression of these symptoms may reduce mortality in COVID-19, cytokine release syndrome, cytokine release syndrome due to CAR-T therapy, sepsis, systemic inflammatory response syndrome, hemophagocytic syndrome, macrophage activation syndrome, influenza, GVHD, systemic vasculitis, Kawasaki disease, Takayasu arteritis, and others.

Cytokine storm is a symptom that also occurs in other diseases; for example, Patent Literature 1 (International Publication WO2016/104436) discloses a cytokine storm inhibitor that contains histidine-rich glycoproteins as active compounds. The disclosed cytokine storm inhibitors have an inhibitory effect on at least two or more cytokines selected from IL-6, TNF-α, and IL-10.

In addition, Patent Literature 2 (JP 2012-20953) discloses prophylactic and/or therapeutic agents for the prevention and/or treatment of fulminant type inflammation containing spiramycin or josamycin (leucomycin A3), or a pharmaceutically acceptable salt thereof, as active compounds. This prophylactic and/or therapeutic agent for the prevention and/or treatment of fulminant inflammation caused by influenza infection (preferably H5N1 or H1N1).

### [Citation List]

### [Patent Literature]

[Patent Literature 1] International Publication WO2016/104436
[Patent Literature 2] JP 2012-20953

### [Non-Patent Literature]

[Non-Patent Literature 1] Hu L, Wu W, Chai X, Luo J, Wu Q.: Bioorg. Med. Chem. Lett. 2011, 21, 4013-4015.

### [Summary of Invention]

### [Technical Problem]

Currently, the number of deaths due to COVID-19 is increasing daily. With such a pandemic increase in the number of patients, the treatment system has not been able to respond in time, resulting in a state of so-called "medical collapse". Under these circumstances, many people die because treatment for COVID-19 does not work, as well as those who die because they do not receive treatment in time for severe cases. In addition, some people die from cytokine storms caused by cytokine release syndrome, cytokine release syndrome, cytokine release syndrome due to CAR-T therapy, sepsis, systemic inflammatory response syndrome, hemophagocytic syndrome, macrophage activation syndrome, influenza, GVHD, systemic vasculitis, Kawasaki disease, Takayasu arteritis, and others.

Thus, compounds that inhibit canonical and non-canonical NF-κB pathway activation by NIK via LPS and others and inhibit the production of inflammatory cytokines such as TNFα and IL-6, inhibit the cytokine storm caused by COVID-19, cytokine release syndrome, cytokine release syndrome due to CAR-T therapy, sepsis, systemic inflammatory response syndrome, hemophagocytic syndrome, macrophage activation syndrome, influenza, GVHD, systemic vasculitis, Kawasaki disease, Takayasu arteritis, and others. Inhibition of cytokine storm is highly expected to avoid acute respiratory distress syndrome, disseminated intravascular coagulation syndrome, acute circulation failure, multi organ failure, and others, and lower the number of deaths.

It would be highly desirable if symptom relief could be achieved not only by transdermal or intravenous administration, which can only be done by a qualified physician, but also by oral administration, especially when the number of patients who cannot receive treatment in the medical field is increasing. Given the urgent need for development, it is also desirable to provide pharmaceutical compositions for cytokine storm inhibition that have little or no potential for side effects.

### [Solution to Problem]

The inventors investigated compounds that inhibit cytokine storm to solve the above problem, and found that compounds with xanthone skeleton (including new compounds) are effective.

More specifically, the pharmaceutical compositions for cytokine storm inhibition of the present invention are characterized in that they contain at least one of the compounds of formulas (1) to (4) as an active ingredient.

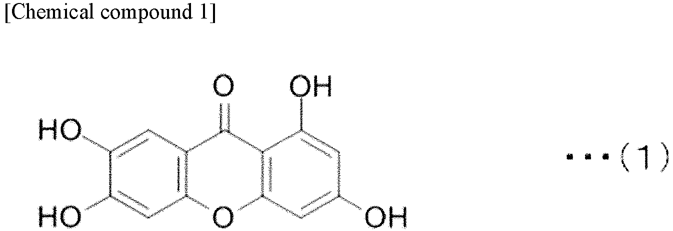

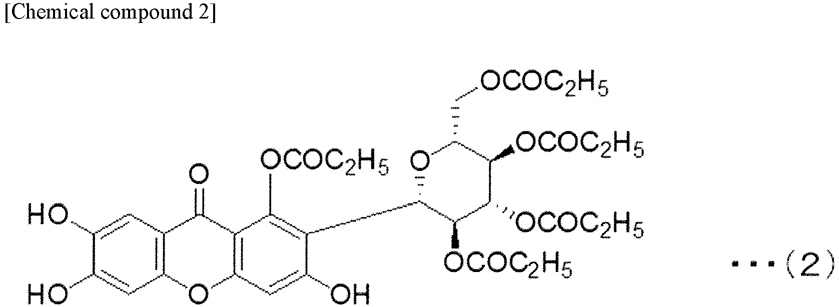

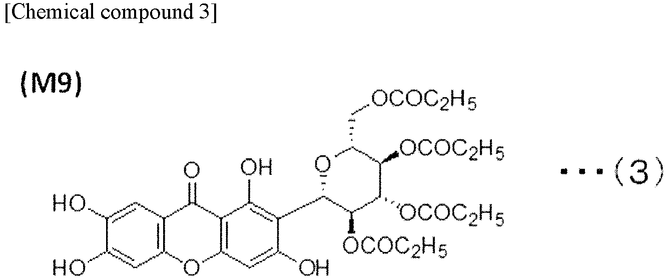

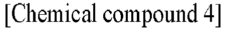

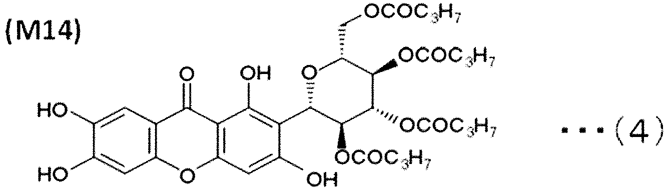

### [Advantageous Effects of Invention]

The pharmaceutical compositions for cytokine storm suppression can provide a therapeutic agent for cytokine storms, one of the causes of death, such as COVID-19, cytokine release syndrome, cytokine release syndrome due to CAR-T therapy, sepsis, systemic inflammatory response syndrome, hemophagocytic syndrome, macrophage activation syndrome, influenza, GVHD, systemic vasculitis, Kawasaki disease, Takayasu Arteritis, etc. In addition, the improvement of cytokine storms caused by COVID-19, cytokine release syndrome, CAR-T therapy, sepsis, systemic inflammatory response syndrome, hemophagocytic syndrome, macrophage activation syndrome, influenza, GVHD, systemic vasculitis, Kawasaki disease, and Takayasu arteritis will improve patient survival. Furthermore, the cytokine storm inhibitors of the present invention can be orally administered, contributing to the improvement of the tight medical field.

### [Brief Description of Drawing]

[Figure 1] The graph shows the inhibitory effect of each compound on LPS-stimulated TNFα mRNA expression in mouse B cells.
[Figure 2] The graph shows the inhibitory effect of each compound on LPS-stimulated IL-6 mRNA expression in mouse B cells.
[Figure 3] The graph shows the inhibitory effect of each compound on LPS-stimulated TNFα mRNA expression in mouse macrophages.
[Figure 4] The graph shows the inhibitory effect of each compound on LPS-stimulated IL-6 mRNA expression in mouse macrophages.
[Figure 5] The graph shows the inhibitory effect of each compound on LPS-stimulated TNFα secretion in mouse B cells.
[Figure 6] The graph shows the inhibitory effect of each compound on LPS-stimulated IL-6 secretion in mouse B cells.
[Figure 7] The graph shows the inhibitory effect of each compound on LPS-stimulated TNFα secretion in mouse macrophages.
[Figure 8] The graph shows the inhibitory effect of each compound on LPS-stimulated IL-6 secretion in mouse macrophages.
[Figure 9] The graph shows the survival rate of mice treated with each compound and LPS after 48 hours.
[Figure 10] The graph shows the amount of TNFα production in the serum of mice treated with each reagent and LPS after 2 hours.
[Figure 11] The graph shows the amount of IL-6 production in the serum of mice treated with each reagent and LPS after 2 hours.

### [Description of Embodiments]

The following is a description of the compounds of the present invention and pharmaceutical compositions for cytokine storm inhibition containing the compounds as active ingredients. The following description is an example of an embodiment and an example of the invention, and the invention is not limited to the following description. The following embodiments may be modified to the extent that they do not depart from the purpose of the invention.

In this description, "improvement" may include not only treatment of the target disease or symptom, but also reduction and suppression of the symptom. In the present invention, cytokine storm suppression is defined as showing an inhibitory effect on the production of TNFα or IL-6.

Compounds pertaining to the present invention are represented by formulas (1) to (4) with a xanthone skeleton. Hereinafter, "compounds pertaining to the present invention" may refer to at least one of the following four compounds.

Formula (1) is 1,3,6,7-tetrahydroxyxanthone (hereafter referred to as "norathyriol"). Norathyriol is a known compound represented by CAS number 3542-72-1.

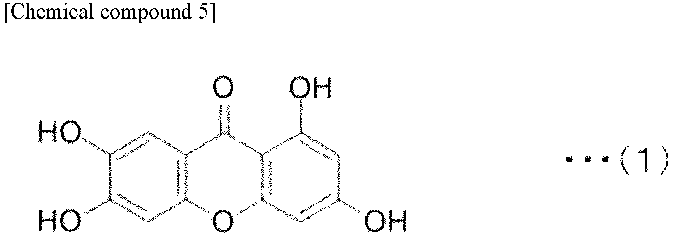

Formula (2) is 1,2',3',4',6'-penta-O-propionylmangiferin (hereafter referred to as "mangiferin 8a").

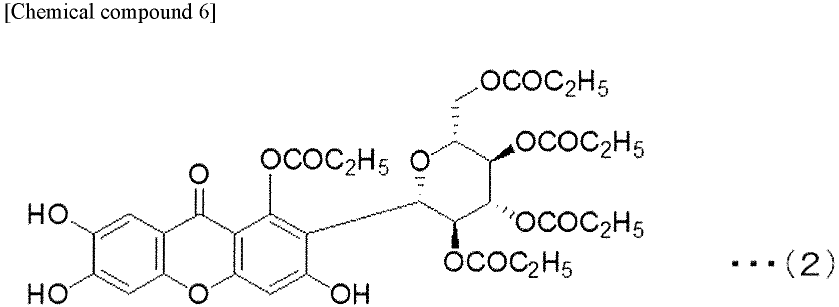

Formula (3) is 2',3',4',6'-tetra-O-propionylmangiferin (hereafter referred to as "M9").

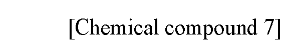

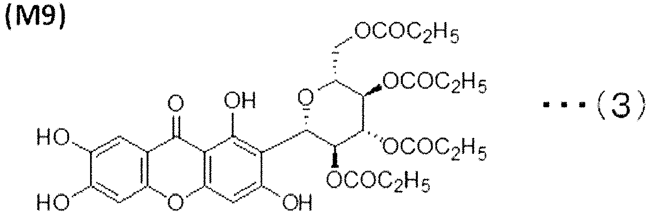

Formula (4) is 2',3',4',6'-tetra-O-butyrylmangiferin (hereafter referred to as "M14").

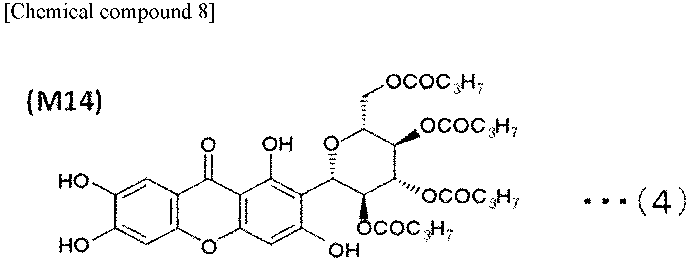

The substitution position numbers for 8a, M9, and M14 were numbered as shown in formula (5).

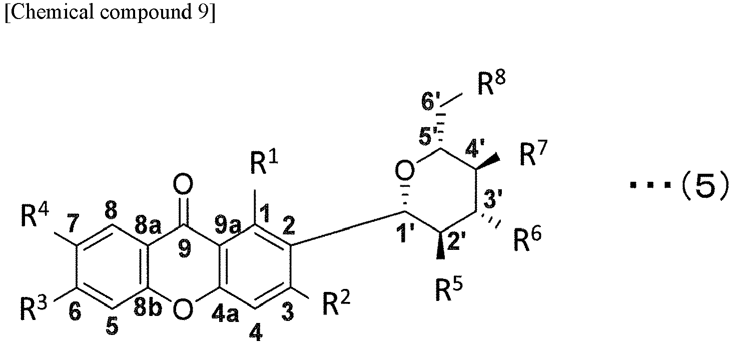

Here, R¹ to R⁸ indicate functional groups.

These compounds of the present invention can be made into pharmaceutical compositions for cytokine storm inhibition. Their mechanism of action is thought to be in the inhibition of NIK in the intracellular transmission pathway and the inhibition of NF-κB from IKK.

Like mangiferin, the compound is a relatively low molecular weight compound that dissolves well in water, can be taken orally.

The pharmaceutical compositions for inhibiting cytokine storms contain at least one of the above four compounds as an active ingredient. It may also contain other ingredients acceptable as drugs.

The pharmaceutical compositions for cytokine storm inhibition can be effective when administered orally. Therefore, they can be provided as an internal drug. For example, pharmacological compositions for cytokine storm inhibition in powder form can be provided in capsules, granules, dispersions, tablets, etc. For oral dosage forms, additives such as binders, lubricants, disintegrants, coloring agents, flavoring substances, preservatives, antioxidants, and stabilizers are added, and capsules, granules, dispersions, and tablets can be manufactured according to conventional methods.

The pharmaceutical compositions for cytokine storm inhibition can be administered by intravenous, subcutaneous, or intramuscular injection. Furthermore, the pharmaceutical compositions of the present invention may be formulated into topical formulations such as liquids, ointments, creams, gels, patchs, and aerosols for parenteral administration. When made into topical formulations, water, lower alcohols, solubilizing agents, surfactants, emulsion stabilizers, gelling agents, adhesives, and other necessary base ingredients can be added. Additives such as vasodilators, adrenal corticosteroids, keratolytic agents, moisturizers, disinfectants, antioxidants, coolants, fragrances, and dyes may also be added as needed.

The compounds of the present invention can also be provided as processed foods or agents. In other words, the compounds of the present invention, when ingested as processed foods, have the same effect as the pharmaceutical compositions for cytokine storm inhibition of the present invention. The agent includes supplements and additives.

Processed foods or agents include not only general processed foods such as candy, gum, jelly, biscuits, cookies, rice crackers, bread, noodles, fish and meat paste products, tea, soft drinks, coffee drinks, milk beverages, lactic acid bacteria beverages, yogurt, ice cream, pudding, and so on, but also foods with health claims, such as food for specified health uses and food with nutrient function claims, as defined by the Ministry of Health, Labour and Welfare's Food with Health Claims System, are included. In addition, nutritional supplements, feed, food additives, etc. are also included in processed foods or agents.

The processed foods can be prepared by adding the above four compounds in the ingredients of these processed foods or agents. The addition of the compounds to the processed foods or agents may be said to be the addition of the compounds as active ingredients.

The processed foods or agents may also be said to be processed foods or agents labeled as being for cytokine storm inhibition.

### [Example]

The synthesis of the compounds shown in formulas (1) through (4) is presented below. The order of synthesis is as follows.
(a) Synthesize intermediate compound M7 from mangiferin.
(b) Synthesize mangiferin 8a (Formula (2)) from intermediate compound M7.
(c) Synthesize M9 (Formula (3)) from mangiferin 8a.
(d) Synthesize intermediate compound M11 from mangiferin.
(e) Synthesize intermediate compound M12 from intermediate compound M11.
(f) Synthesize M14 (Formula 4) from intermediate compound M12.
(g) Synthesize norathyriol (Formula 1).

### < Synthesis of intermediate compound M7 >

Mangiferin (4.21 g, 10.0 mmol), propionic anhydride (19.2 mL, 149 mmol), DMAP (122 mg, 1 mmol) and dry pyridine (60 mL) were heated at 100 °C for 24 h. The reaction solution was poured into ice water (600 mL) and extracted with ethyl acetate. The ethyl acetate layer was washed sequentially with ice-cold 10% sulfuric acid, saturated sodium bicarbonate solution, and saturated brine, dried over anhydrous sodium sulfate, filtered, and concentrated. The residue was purified using column chromatography (n-hexane/ethyl acetate = 1/1) to afford the title compound (7.19 g, 83%) as a colorless solid.

The NMR spectrum is shown below.

IR (KBr): 1774, 1755, 1667, 1620, 1458, 1354, 1273, 1157, 1114, 1083 cm⁻¹. ¹H NMR (800 MHz, DMSO-d₆, 25 °C) δ: 0.71- 0.77/0.93-0.97 (each 3H, m, COCH₂CH₃), 0.98-1.02/1.12-1.16/1.19-1.28 (each 6H, m, COCH₂CH₃), 1.92-2.01 (2H, m, COCH₂CH₃), 2.17-2.32 (6H, m, COCH₂CH₃), 2.60-2.94 (8H, m, COCH₂CH₃), 3.86-3.92 (1H, m, H-6'a), 4.21-4.30 (2H, m, H-5' and H-6'b), 5.01-5.06 (1H, m, H-4'), 5.10-5.20 (1H, m, H-1'), 5.45-5.53 (2H, m, H-2' and H-3'), 7.51/7.53 (1H, each s, H-4), 7.68/7.69 (1H, each s, H-5), 7.97/7.98 (1H, each s, H-8). ¹³C NMR (200 MHz, DMSO-d₆ 25 °C) δ: 8.64/8.75/8.77/8.91/8.94/8.96/9.00/9.13/9.16 (COCH₂CH₃), 26.58/26.63/26.7/26.8/26.89/26.94/27 0/27.1/27.32/27.34 (COCH₂CH₃), 62.1/62.2 (C6'), 68.18/68.20 (C4'), 69.6/70.0 (C2'), 70.8/71.3 (C1'), 73.5/73.6 (C3'), 75.0/75.1 (C5'), 110.2/111.8 (C4), 111.6/112.7 (C9a), 113.2/113.3 (C5), 118.8/118.9 (C2), 119.6/119.7 (C8a), 120.3/120.4 (C8), 139.5/139.6 (C7), 147.9 (C6), 149.1/150.9 (C1), 152.47/152.51 (C8b), 153.4/154.8 (C4a), 156.5/156.8 (C3), 170.9/171.04/171.06/171.09/171.67/171.72/171.9/172.4/172.6/172.7/173.18/173.22/173.51/173.54 (COCH₂CH₃), 173.1 (C9). HRMS (ESI) m/z: [M+Na]⁺ Calcd for C₄₃H₅₀O₁₉Na 893.2839; Found 893.2853.

The structure of intermediate compound M7 is shown in formula (6).

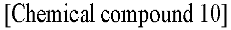

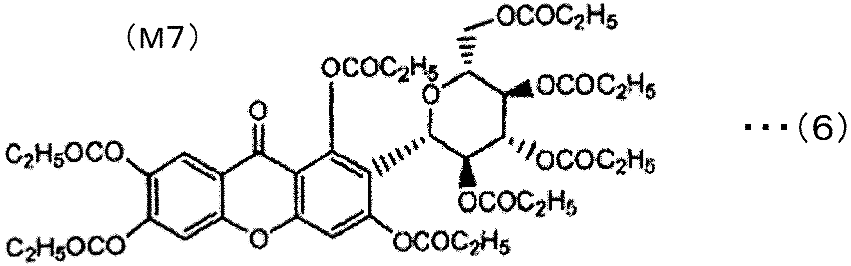

### < Synthesis of (2) formula (mangiferin 8a) >

Mangiferin 8a was synthesized as follows: A mixture of 1,3,2',3',4',6,6',7-octa-O-propionylmangiferin (M7) (5.06 g, 5.68 mmol), ammonium acetate (6.7 g, 87.0 mmol), methanol (160 mL) and water (20 mL) was stirred at room temperature for 6 h. Methanol was removed from the reaction solution by reduced pressure concentration, and the residue was diluted with ethyl acetate (100 mL). The mixture was washed sequentially with water and saturated brine, dried over anhydrous sodium sulfate, filtered, and concentrated. The residue was purified by column chromatography [CHCl₃/CH₃OH (20:1)] to afford the title compound (3.89 g, 95%) as a pale yellow solid. Mangiferin 8a is represented by formula (2).

The NMR spectrum is shown below.

IR (KBr): 3406, 1751, 1620, 1462, 1354, 1284, 1192, 1083 cm⁻¹. ¹H NMR (800 MHz, DMSO-d₆, 25 °C) δ: 0.74/0.94 (each 3H, t-like, J= 7.6, COCH₂CH₃), 0.97-1.02 (6H, m, COCH₂CH₃), 1.20/1.23 (each 1.5H, t-like, J = 7.6, COCH₂CH₃), 1.92-2.02 (2H, m, COCH₂CH₃), 2.15-2.33 (6H, m, COCH₂CH₃), 2.61-2.86 (2H, m, COCH₂CH₃), 3.85 (0.5H, dd-like, J = ca. 12.5, 1.9, H-6a'), 4.05 (0.5H, br d-like, J = ca. 12.5, H-6a'), 4.07 (0.5H, ddd, J = 9.8, 4.7, 1.9, H-5'), 4.09-4.14 (0.5H, m, H-6b'), 4.11 (0.5H, br d-like, J = ca. 9.5, H-5'), 4.24 (0.5H, dd, J = 12.5, 4.7, H-6b'), 4.96 (0.5H, d, J = 9.9, H-1'), 5.00 (0.5H, dd, J = 9.5, 9.5, H-4'), 5.02 (0.5H, dd, J = 9.8, 9.8, H-4'), 5.16 (0.5H, d, J = 10.0, H-1'), 5.35 (0.5H, dd, J = 9.5, 9.5, H-3'), 5.40 (0.5H, dd, J = 9.8, 9.5, H-3'), 5.49 (0.5H, dd, J = 10.0, 9.5, H-2'), 5.84 (0.5H, dd, J = 9.9, 9.5, H-2'), 6.72/6.75 (each 0.5H, s, H-4), 6.80/6.81 (each 0.5H, s, H-5), 7.29/7.30 (each 0.5H, s, H-8), 9.62/10.5 (each 1H, br s, OH), 11.2/11.4 (each 0.5H, br s, OH). ¹³C NMR (200 MHz, DMSO-d₆, 25 °C) δ: 8.81/8.86/8.96/9.02/9.06/9.11/9.12/9.20 (COCH₂CH₃), 26.6/26.89/26.91/26.94/27.00/27.04/27.1/27.4 (COCH₂CH₃), 62.1/62.3 (C-6'), 68.2/68.3 (C-4'), 68.7/70.4 (C-2'), 71.0/71.1 (C-1'), 73.8/74.1 (C-3'), 74.6/75.2 (C-5'), 99.5/100.8 (C-4), 102.5 (C-5), 106.6/107.8 (C-9a), 109.1 (C-8), 113.3 (C-2), 114.0/114.1 (C-8a), 143.8 (C-7), 149.77/149.82 (C-6, C-1), 151.3 (C-1), 153.3 (C-8b), 157.5/157.7 (C-4a), 160.7/162.2 (C-3), 171.1/171.9/172.2/172.5/172.8/173.1/173.4/173.6 (COCH₂CH₃), 172.68/172.71 (C-9). HRMS (ESI) m/z: [M-H]⁻ Calcd for C₃₄H₃₇O₁₆ 701.2076; Found 701.2070.

### < Synthesis of (3) formula (M9) >

A mixture of 1,2',3',4',6-penta-O-propionylmangiferin (mangiferin 8a) (3.88 g, 5.52 mmol), N,N-dimethyltrimethylenediamine (3.47 mL, 27.6 mmol) and DMSO (40 mL) was stirred at room temperature for 1 hour. The reaction solution was poured into ice water (500 mL) and extracted with a mixture of diethyl ether and hexane (3/1). The organic layer was washed with saturated brine, dried over anhydrous sodium sulfate, filtered, and concentrated. The residue was purified by column chromatography [CHCl₃/CH₃OH (20:1)] to afford the title compound (3.08 g, 86%) as a pale yellow solid. The structure of M9 is represented by formula (3).

The NMR spectrum is shown below.

IR (KBr): 3399, 1751, 1618, 1475, 1292, 1190, 1084 cm⁻¹. ¹H NMR (800 MHz, DMSO-d₆, 60 °C) δ: 0.74/0.97/1.02/1.03 (each 3H, t, J= 7.6, COCH₂CH₃), 1.95/1.98 (each 1H, dq, J = 16.5, 7.6, COCH₂CH₃), 2.18 (2H, q, J = 7.6, COCH₂CH₃), 2.22-2.34 (4H, m, COCH₂CH₃), 3.99 (1H, ddd-like, J = 9.8, 4.5, 2.5, H-5'), 4.10 (1H, dd, J = 12.5, 2.5, H-6a'), 4.12 (1H, dd, J = 12.5, 4.5, H-6b'), 5.06 (1H, dd, J = 9.8, 9.8, H-4'), 5.10 (1H, d, J = 10.0, H-1'), 5.31 (1H, dd, J = 9.8, 9.5, H-3'), 5.85 (1H, br dd, J = 10.0, 9.5, H-2'), 6.35 (1H, s, H-4), 6.85 (1H, s, H-4), 7.34 (1H, s, H-8), 9.0-11.0 (3H, br, OH), 13.9 (1H, s, OH). ¹³C NMR (200 MHz, DMSO-d₆, 60 °C) δ: 8.71/8.79/8.82 (COCH₂CH₃), 26.72/26.73/26.77/26.83 (COCH₂CH₃), 62.0 (C-6'), 68.3 (C-4'), 69.1 (C-2'), 70.4 (C-1'), 74.2 (C-3'), 75.0 (C-5'), 93.3 (C-4), 101.0 (C-9a), 102.6 (C-5), 104.2 (C-2), 108.2 (C-8), 111.7 (C-8a), 143.8 (C-7), 150.8 (C-6), 154.2 (C-8b), 156.8 (C-4a), 161.9 (C-1), 163.6 (C-3), 171.9/172.5/172.7/173.1 (COCH₂CH₃), 179.0 (C-9). HRMS (ESI) m/z: [M-H]⁻ Calcd for C₃₁H₃₃O₁₅ 645.1814; Found 645.1817.

### < Synthesis of intermediate compound M11 >

Intermediate compound M11 was synthesized according to the method for intermediate compound M7, except that propionic anhydride was replaced with butyric anhydride. The yield was 81%.

The NMR spectrum is shown below.

Colorless solid: IR (KBr): 1775, 1751, 1665, 1618, 1458, 1157, 1092 cm⁻¹. ¹H NMR (800 MHz, DMSO-d₆, 25 °C) δ: 0.52-1.11 (24H, m, COCH₂CH₂CH₃), 1.20-1.97 (16H, m, COCH₂CH₂CH₃), 2.14-2.88 (16H, m, COCH₂CH₂CH₃), 3.88-3.94 (1H, m, H -6'a), 4.16-4.25 (2H, m, H-5' and H-6'b), 5.02-5.06 (1H, m, H-4'), 5.06-5.12 (1H, m, H-1'), 5.45-5.52 (1H, m, H-3'), 5.54-5.65 (1H, m, H-2'), 7.50/7.53 (1H, each s, H-4), 7.68/7.69 (1H, each s, H-5), 7.95/7.96 (1H, each s, H-8). ¹³C NMR (200 MHz, DMSO-d₆, 25°C) δ: 13.1/13.41/13.45/13.48/13.51/13.58/13.64/13.8 (COCH₂CH₂CH₃), 17.5/17.62/17.66/17.72/17.76/17.83/17.88/17.90 (COCH₂CH₂CH₃), 34.9/35.09/35.15/35.17/35.19/35.31/35.33/35.4/35.68/35.73 (COCH₂CH₂CH₃), 62.0/62.2 (C6'), 68.2/68.3 (C4'), 69.3/69.8 (C2'), 70.9/71.3 (C1'), 73.36/73.43 (C3'), 75.1/75.2 (C5'), 110.2/111.6 (C4), 111.6/112.6 (C9a), 113.3/113.4 (C5), 118.7/118.8 (C2), 119.6/119.7 (C8a), 120.35/120.40 (C8), 139.5 (C7), 147.82/147.85 (C6), 149.1/150.8 (C1), 152.46/152.49 (C8b), 153.4/154.7 (C4a), 156.6/156.8 (C3), 169.9/170.14/170.17/170.18/170.7/170.8/171.0/171.4/171.6/171.8/172.0/172.1/172.5/172.6 (COCH₂CH₃), 173.1 (C9).HRMS (ESI) m/z: [M+Na]⁺ Calcd for C₅₁H₆₇O₁₉Na 1005.4091; Found 1005.4092.

The structure of intermediate compound M11 is shown in formula (7).

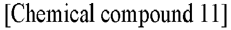

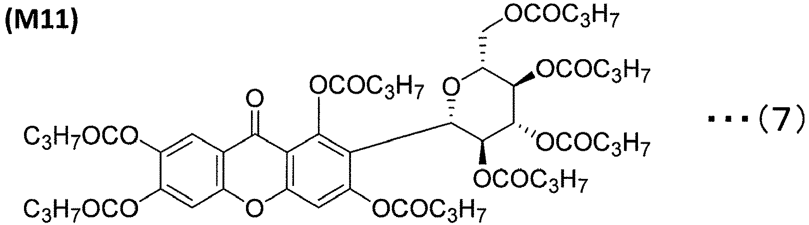

### < Synthesis of intermediate compound M12 >

In the synthetic method of mangiferin 8a, 1,3,2',3',4',6,6',7-octa-O-propionylmangiferin (M7) was replaced by 1,3,2',3',4',6,6',7-octa-O-butyrylmangiferin (M11) was used as the starting material, and the synthesis of intermediate compound M12 was carried out according to the synthetic method of mangiferin 8a. The yield was 93%.

The NMR spectrum is shown below.

Pale brown solid: IR (KBr): 3385, 1751, 1624, 1458, 1288, 1188, 1099 cm⁻¹. ¹H NMR (800 MHz, DMSO-d⁶, 25 °C) δ: 0.48-1.09 (15H, m, COCH₂CH₂CH₃), 1.18-1.83 (10H, m, COCH₂CH₂CH₃), 1.89-2.86 (10H, m, COCH₂CH₂CH₃), 3.87 (0.5H, dd-like, J = ca. 12.6, 1.6, H-6a'), 4.02-4.11 (2H, br m, H-5, H-5', H-6a', H-6b'), 4.16 (0.5H, dd, J = 12.6, 4.8, H-6b'), 4.90 (0.5H, d, J = 9.6, H-1'), 5.01 (0.5H, dd, J = 9.6, 9.6, H-4'), 5.02 (0.5H, dd, J = 9.6, 9.6, H-4'), 5.15 (0.5H, d, J = 10.4, H-1'), 5.35 (0.5H, dd, J = 9.6, 9.6, H-3'), 5.37 (0.5H, br dd-like, J = ca. 9.6, 9.6, H-3'), 5.54 (0.5H, dd, J = 10.4, 9.6, H-2'), 5.88 (0.5H, br dd-like, J = ca. 9.6, 9.6, H-2'), 6.72/6.74 (each 0.5H, s, H-4), 6.797/6.803 (each 0.5H, s, H -5), 7.29/7.30 (each 0.5H, s, H-8), 9.67/10.5/11.2 (each 1H, br s, OH). ¹³C NMR (200 MHz, DMSO-d₆, 25°C) δ: 13.1/13.4/13.50/13.54/13.6/13.7/13.9 (COCH₂CH₂CH₃), 17.7/17.90/17.93/17.96/18.02 (COCH₂CH₂CH₃), 35.1/35.2/35.3/35.37/35.41/35.46/35.50/35.54/35.9 (COCH₂CH₂CH₃), 62.0/62.4 (C-6'), 68.2/68.4 (C-4'), 68.5/70.3 (C-2'), 71.1/71.2 (C-1'), 73.7/74.0 (C-3'), 74.9/75.3 (C-5'), 99.7/100.8 (C-4), 102.52/102.54 (C-5), 106.6/107.8 (C-9a), 109.1 (C-8), 113.2/113.4 (C-2), 114.06/114.08 (C-8a), 143.9 (C-7), 149.8/149.9 (C-6, C-1), 151.3 (C-1), 153.3 (C-8b), 157.6/157.7 (C-4a), 160.9/162.3 (C-3), 170.3/171.1/171.2/171.5/171.9/172.2/172.6 (COCH₂CH₃), 172.7 (C-9). HRMS (ESI) m/z: [M-H]⁻ Calcd for C₃₉H₄₇O₁₆ 771.2859; Found 771.2858. The structure of intermediate compound M12 is shown in formula (8).

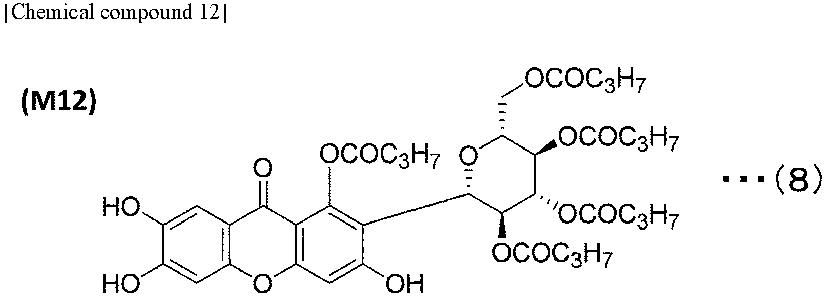

### < Synthesis of (4) formula (M14) >

In the synthetic method of M9, 1,3,2',3',4',6,6',7-octa-O-propionylmangiferin (M7) was replaced by 1,2',3',4',6-penta-O-butyrylmangiferin (M12) was used as the starting material, and the synthesis of M14 was carried out according to the synthetic method of M9. The yield was 84%.

The NMR spectrum is shown below.

Pale yellow solid: IR (KBr): 3399, 1751, 1618, 1474, 1292, 1188, 1085 cm⁻¹. ¹H NMR (800 MHz, DMSO-d⁶, 60°C) δ: 0.50 (3H, br, t-like, J = ca. 7.0, COCH₂CH₂CH₃), 0.83/0.871/0.872 (each 3H, t, J = 7.4, COCH₂CH₂CH₃), 1.18-1.30/1.44-1.49 (each 2H, m, COCH₂CH₂CH₃), 1.50-1.56 (6H, m, COCH₂CH₂CH₃), 1.93 (2H, t, J = 7.0, COCH₂CH₂CH₃), 2.15 (2H, t, J = 7.2, COCH₂CH₂CH₃), 2.22-2.31 (4H, m, COCH₂CH₂CH₃), 3.98 (1H, ddd-like, J = 9.8, 4.6, 2.5, H-5'), 4.09 (1H, dd, J = 12.5, 4.6, H-6a'), 4.11 (1H, dd, J = 12.5, 2.5, H-6b'), 5.06 (1H, dd, J = 9.8, 9.8, H-4'), 5.09 (1H, d, J = 10.0, H-1'), 5.31 (1H, dd, J = 9.8, 9.5, H-3'), 5.85 (1H, br dd, J = 10.0, 9.5, H-2'), 6.34 (1H, s, H-4), 6.85 (1H, s, H-4), 7.38 (1H, s, H-8), 8.9-11.5 (3H, br, OH), 13.9 (1H, s, OH). ¹³C NMR (200 MHz, DMSO-d₆, 60°C) δ: 12.6/13.13/13.16/13.22 (COCH₂CH₂CH₃), 17.62/17.64/17.66 (COCH₂CH₂CH₃), 35.16/35.23/35.26/35.31 (COCH₂CH₂CH₃), 61.9 (C-6'), 68.3 (C-4'), 69.0 (C-2'), 70.4 (C-1'), 74.1 (C-3'), 75.0 (C-5'), 93.3 (C-4), 101.1 (C-9a), 102.6 (C-5), 104.2 (C-2), 108.2 (C-8), 111.7 (C-8a), 143.8 (C-7), 150.8 (C-6), 154.2 (C-8b), 156.8 (C-4a), 162.1 (C-1), 163.7 (C-3), 170.9/171.6/171.7/172.3 (COCH₂CH₃), 179.0 (C-9). HRMS (ESI) m/z: [M-H]⁻ Calcd for C₃₅H₄₁O₁₅ 701.2440; Found 701.2440.

### < Synthesis of (1) formula (norathyriol) >

Norathyriol was synthesized according to the method of Non-Patent Literature 1. Norathyriol is a known substance represented by CAS number 3542-72-1 and is commercially available and may be purchased.

Norathyriol was synthesized as follows. Namely, 2,4,5-trimethoxybenzoic acid (Compound II) was treated with thionyl chloride to obtain 2,4,5-trimethoxybenzoic acid chloride (Compound III), according to the method described in the literature (Non-Patent Literature 1). Next, the obtained compound (Compound III) was reacted with 1,3,5-trimethoxybenzene (Compound IV) by Friedel-Craft reaction to give 2-hydrodoxy-2',4,4',5,6'-pentamethoxybenzophenone (Compound V) was obtained. The compound (V) was further treated with tetrabutylammonium hydroxide to afford 1,3,6,7-tetramethoxyxanthone (compound VI), followed by demethylation to give norathyriol (compound I) in 39% yield.

The following is a detailed description of the synthetic route of this example.

### (1) Synthesis of 2,4,5-trimethoxybenzoic acid chloride (Compound III)

2,4,5-trimethoxybenzoic acid (Compound II, 8.49 g, 0.040 mol) was dissolved by gradually adding thionyl chloride (5 mL) at room temperature under an argon atmosphere, and then heated reflux for 6 h. After completion of the reaction, the reaction mixture was distilled off under reduced pressure to obtain 2,4,5-trimethoxybenzoic acid chloride (compound III, 8.30 g, 90%). The obtained compound (III) was immediately used for the next reaction.

### (2) Synthesis of 2-hydrodoxy-2',4,4',5,6'-pentamethoxybenzophenone (Compound V)

To a mixed suspension of 2,4,5-trimethoxybenzoic acid chloride (Compound III, 8.07 g, 0.035 mol) obtained as described above, 1,3,5-trimethoxybenzene (Compound IV, 6.48 g, 0.0385 mol), and anhydrous diethyl ether (500 mL) was added aluminum chloride (16 g) gradually at room temperature under argon atmosphere, and the reaction mixture was stirred at room temperature for 48 h. After removing the solvent from the reaction mixture under reduced pressure, water was added to the residue and extracted with ethyl acetate. The extract was washed with saturated brine, dried over anhydrous sodium sulfate, filtered off the drying agent using folded filter paper, and the filtrate was removed under reduced pressure to give the crude product. The crude product was purified by silica gel column chromatography (n-hexane:ethyl acetate = 1:1, v/v) to give 2-hydrodoxy-2',4,4',5,6'-pentamethoxybenzophenone (Compound V, 8.43 g, 69%).

### (3) Synthesis of 1,3,6,7-tetramethoxyxanthone (Compound VI)

2-hydrodoxy-2',4,4',5,6'-pentamethoxybenzophenone (Compound V, 6.97 g, 0.020 mol) obtained as described above was dissolved in a mixture of pyridine (10 mL) and water (10 mL), and 40% tetrabutylammonium hydroxide solution (5 mL) was added and heated to reflux for 6 h. The resulting reaction mixture was poured into 5% hydrochloric acid and extracted with ethyl acetate. The extract was washed with saturated brine, dried over anhydrous sodium sulfate, filtered off the drying agent using folded filter paper, and the filtrate was removed under reduced pressure. The crude product was purified by silica gel column chromatography (n-hexane: ethyl acetate = 1:1, v/v) to afford 1,3,6,7-tetramethoxyxanthone (compound VI, 5.82 g, 92%).

### (4) Synthesis of norathyriol (Compound I)

A mixture of 1,3,6,7-tetramethoxyxanthone (Compound VI, 4.74 g, 0.015 mol) and pyridine hydrochloride (5.00 g) obtained as described above was heated and stirred at 200 °C for 6 h. The resulting reaction mixture was allowed to cool to room temperature, poured into 5% hydrochloric acid, and extracted with ethyl acetate. The extract was washed with saturated brine, dried over anhydrous sodium sulfate, filtered off the drying agent using folded filter paper, and the filtrate was removed under reduced pressure to obtain the crude product. The crude product was purified by silica gel column chromatography (chloroform:methanol = 7:1, v/v) to afford norathyriol of formula (1) (Compound I, 2.65 g, 68%).

The following examples are provided to demonstrate the cytokine storm inhibitory effect of the compounds of the present invention.

### < Example 1 > Suppression of TNFα mRNA expression in B cells

The inhibitory effect of compounds on TNFα mRNA expression involved in cytokine storm was examined using mouse B cells.

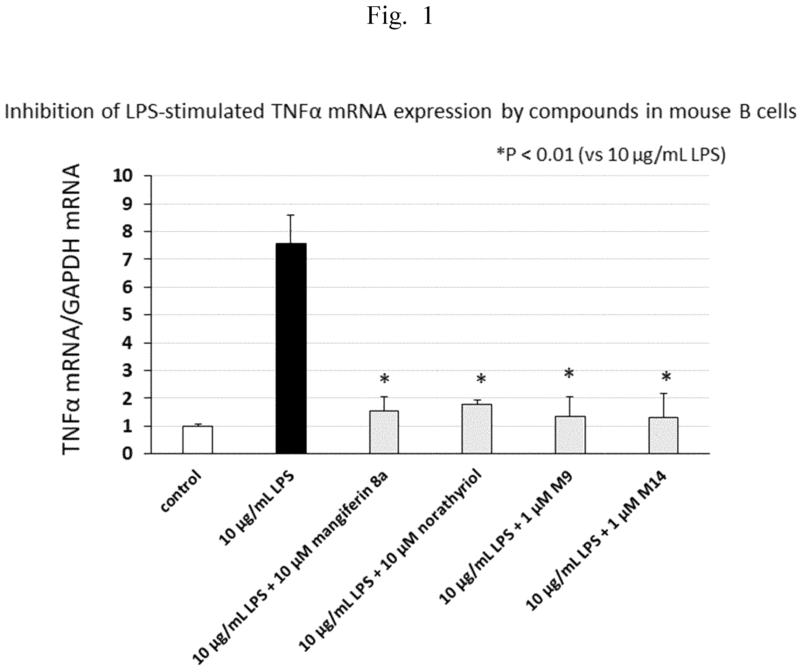

Mouse B cells were pre-cultured with RPMI1640 medium for 1 day. After incubation, mouse B cells were treated with 10 µM mangiferin 8a, 10 µM norathyriol, 1 µM M9, and 1 µM M14 for 1 day. Then, 10 µg/mL LPS was added to mouse B cells and cultured for 4 h. After incubation, cells were collected, RNA was extracted, and TNFα mRNA expression was measured by real time PCR. The results are shown in Figure 1.

Referring to Figure 1, the horizontal axis shows the treatment of each compound, and the vertical axis shows the ratio of TNFα mRNA to mRNA of the housekeeping gene glyceraldehyde 3-phosphate dehydrogenase (GAPDH). For controls, only 0.5% DMSO (diluted in PBS) used to dissolve the compounds was added. 10 µg/mL LPS administered is called the LPS-treated group, LPS and mangiferin 8a administered in combination is called the mangiferin 8a-treated group, LPS and norathyriol administered in combination is called the norathyriol group, LPS and M9 administered in combination is called the M9-treated group, LPS and M14 administered in combination is called the M14-treated group, respectively.

Referring to Figure 1, the LPS-treated group showed increased expression of TNFα mRNA. This is a reproduction of the state of cytokine storm. On the other hand, a decrease in LPS-induced TNFα mRNA expression was observed in the mangiferin 8a-, norathyriol-, M9-, and M14-treated groups.

### < Example 2 > Suppression of IL-6 mRNA expression in B cells

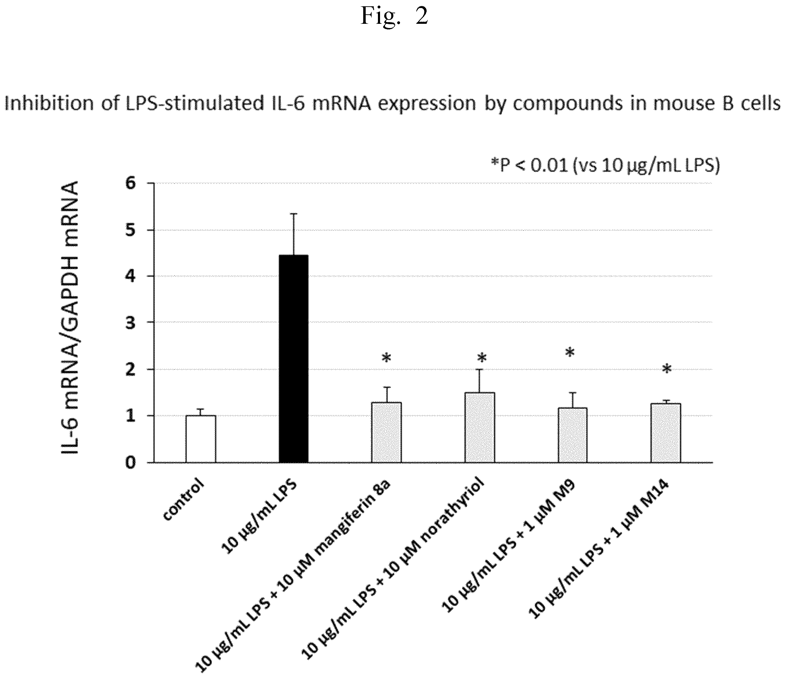

The inhibitory effect of compounds on IL-6 mRNA expression involved in cytokine storm was examined using mouse B cells. The experimental procedure is the same as in Example 1. The results are shown in Figure 2.

Referring to Figure 2, the horizontal axis shows the treatment of each compound, and the vertical axis shows the ratio of IL-6 mRNA to mRNA of the housekeeping gene GAPDH. For controls, only 0.5% DMSO (diluted in PBS) used to dissolve the compounds was added. 10 µg/mL LPS administered is called the LPS-treated group, LPS and mangiferin 8a administered in combination is called the mangiferin 8a-treated group, LPS and norathyriol administered in combination is called the norathyriol group, LPS and M9 administered in combination is called the M9-treated group, LPS and M14 administered in combination is called the M14-treated group, respectively.

Referring to Figure 2, the LPS-treated group showed increased expression of IL-6 mRNA. This is a reproduction of the state of cytokine storm. On the other hand, a decrease in LPS-induced IL-6 mRNA expression was observed in the mangiferin 8a-, norathyriol-, M9-, and M14-treated groups.

### < Example 3 > Suppression of TNFα mRNA expression in macrophages

The inhibitory effect of compounds on TNFα mRNA expression involved in cytokine storm was examined using mouse macrophages.

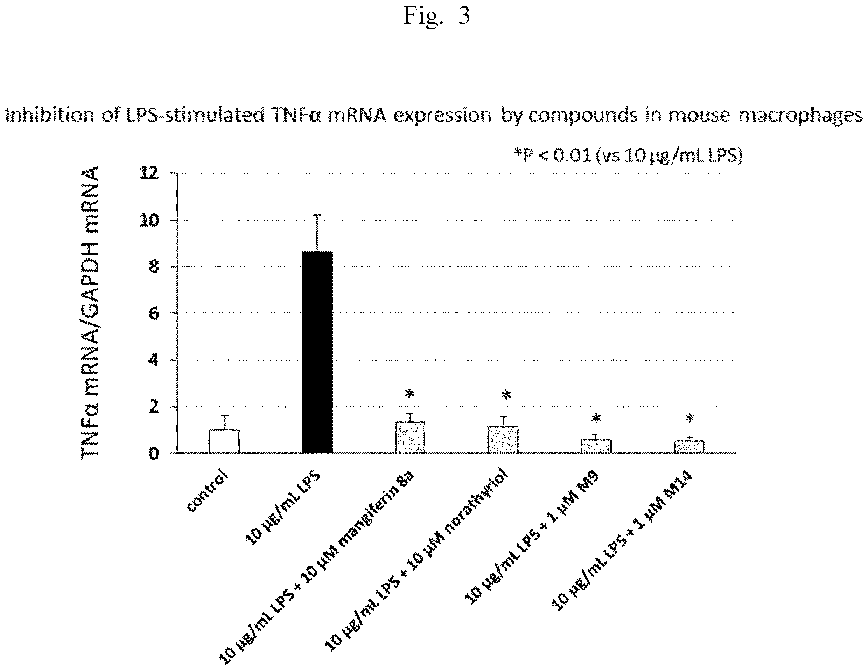

Mouse macrophages were pre-cultured with RPMI1640 medium for 1 day. After incubation, mouse macrophages were treated with 10 µM mangiferin 8a, 10 µM norathyriol, 1 µM M9, and 1 µM M14 for 1 day. Then, 10 µg/mL LPS was added to mouse macrophages and cultured for 4 h. After incubation, cells were collected, RNA was extracted, and TNFα mRNA expression was measured by real time PCR. The results are shown in Figure 3.

Referring to Figure 3, the horizontal axis shows the treatment of each compound, and the vertical axis shows the ratio of TNFα mRNA to mRNA of the housekeeping gene glyceraldehyde 3-phosphate dehydrogenase (GAPDH). For controls, only 0.5% DMSO (diluted in PBS) used to dissolve the compounds was added. 10 µg/mL LPS administered is called the LPS-treated group, LPS and mangiferin 8a administered in combination is called the mangiferin 8a-treated group, LPS and norathyriol administered in combination is called the norathyriol group, LPS and M9 administered in combination is called the M9-treated group, LPS and M14 administered in combination is called the M14-treated group, respectively.

Referring to Figure 3, the LPS-treated group showed increased expression of TNFα mRNA. This is a reproduction of the state of cytokine storm. On the other hand, a decrease in LPS-induced TNFα mRNA expression was observed in the mangiferin 8a-, norathyriol-, M9-, and M14-treated groups.

### < Example 4 > Suppression of IL-6 mRNA expression in macrophages

The inhibitory effect of compounds on IL-6 mRNA expression involved in cytokine storm was examined using mouse macrophages.

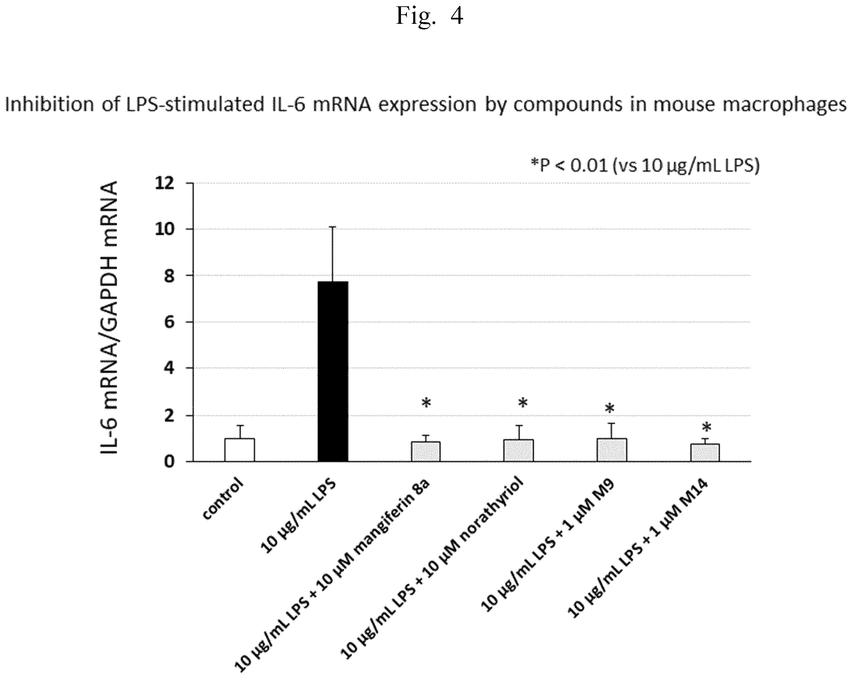

The experimental procedure is the same as in Example 3. The results are shown in Figure 4. Referring to Figure 4, the horizontal axis shows the treatment of each compound, and the vertical axis shows the ratio of IL-6 mRNA to mRNA of the housekeeping gene GAPDH. For controls, only 0.5% DMSO (diluted in PBS) used to dissolve the compounds was added. 10 µg/mL LPS administered is called the LPS-treated group, LPS and mangiferin 8a administered in combination is called the mangiferin 8a-treated group, LPS and norathyriol administered in combination is called the norathyriol group, LPS and M9 administered in combination is called the M9-treated group, LPS and M14 administered in combination is called the M14-treated group, respectively.

Referring to Figure 4, the LPS-treated group showed increased expression of IL-6 mRNA. This is a reproduction of the state of cytokine storm. On the other hand, a decrease in LPS-induced IL-6 mRNA expression was observed in the mangiferin 8a-, norathyriol-, M9-, and M14-treated groups.

### < Example 5 > Suppression of TNFα production in B cells

The inhibitory effect of compounds on TNFα production involved in cytokine storm was examined using mouse B cells.

Mouse B cells were pre-cultured with RPMI1640 medium for 1 day. After incubation, mouse B cells were treated with 10 µM mangiferin 8a, 10 µM norathyriol, 1 µM M9, and 1 µM M14 for 1 day. Then, 10 µg/mL LPS was added to mouse B cells and cultured for 2 days. After incubation, the culture supernatant was collected and TNFα production was determined by enzyme-linked immunosorbent assay (ELISA). This measurement was performed using a mouse TNFα ELISA kit (R&D).

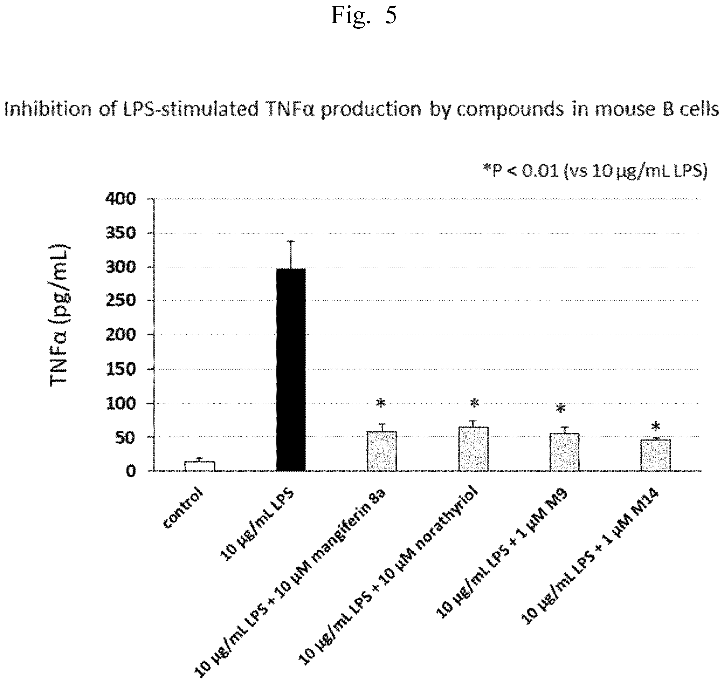

Results are shown in Figure 5. The horizontal axis represents the sample group and the vertical axis represents TNFα production. In control, TNFα was 15.13 pg/mL, whereas in 10 µg/mL LPS, TNFα was 296.31 pg/mL, indicating that LPS stimulation activated B cells and enhanced TNFα secretion. On the other hand, the mangiferin 8a-treated group, norathyriol-treated group, M9-treated group, and M14-treated group, 59.31 pg/mL, 64.13 pg/mL, 55.13 pg/mL, and 46.13 pg/mL, respectively.

As described above, the mangiferin 8a-, norathyriol-, M9-, and M14-treated groups significantly suppressed TNFα production in LPS stimulation.

### < Example 6 > Suppression of IL-6 production in B cells

The inhibitory effect of compounds on IL-6 production involved in cytokine storm was examined using mouse B cells.

Mouse B cells were pre-cultured with RPMI1640 medium for 1 day. After incubation, mouse B cells were treated with 10 µM mangiferin 8a, 10 µM norathyriol, 1 µM M9, and 1 µM M14 for 1 day. Then, 10 µg/mL LPS was added to mouse B cells and cultured for 2 days. After incubation, the culture supernatant was collected and IL-6 production was determined by enzyme-linked immunosorbent assay (ELISA). This measurement was performed using a mouse IL-6 ELISA kit (R&D).

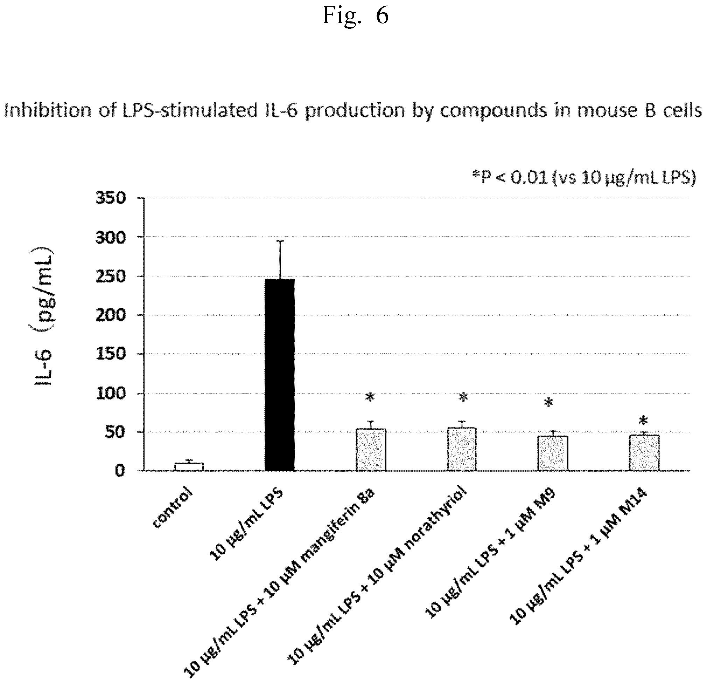

Results are shown in Figure 6. The horizontal axis represents the sample group and the vertical axis represents IL-6 production. In control, IL-6 was 9.46 pg/mL, whereas in 10 µg/mL LPS, IL-6 was 246.13 pg/mL, indicating that LPS stimulation activated B cells and enhanced IL-6 secretion. On the other hand, the mangiferin 8a-treated group, norathyriol-treated group, M9-treated group, and M14-treated group, 54.13 pg/mL, 56.13 pg/mL, 45.12 pg/mL, and 45.13 pg/mL, respectively.

As described above, the mangiferin 8a-, norathyriol-, M9-, and M14-treated groups significantly suppressed IL-6 production in LPS stimulation.

### < Example 7 > Suppression of TNFα production in macrophages

The inhibitory effect of compounds on TNFα production involved in cytokine storm was examined using mouse macrophages.

Mouse macrophages were pre-cultured with RPMI1640 medium for 1 day. After incubation, mouse macrophages were treated with 10 µM mangiferin 8a, 10 µM norathyriol, 1 µM M9, and 1 µM M14 for 1 day. Then, 10 µg/mL LPS was added to mouse macrophages and cultured for 2 days. After incubation, the culture supernatant was collected and TNFα production was determined by enzyme-linked immunosorbent assay (ELISA). This measurement was performed using a mouse TNFα ELISA kit (R&D).

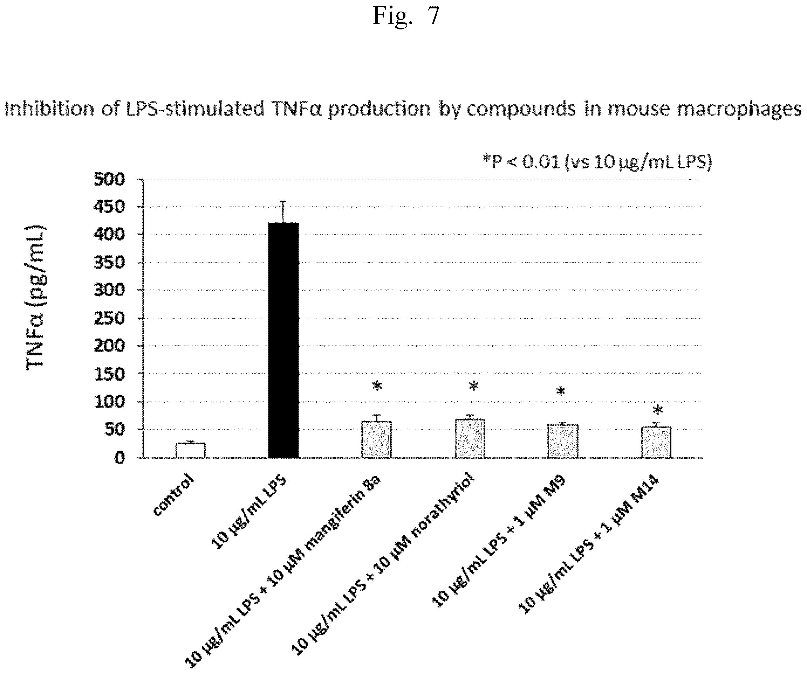

Results are shown in Figure 7. The horizontal axis represents the sample group and the vertical axis represents TNFα production. In control, TNFα was 24.11 pg/mL, whereas in 10 µg/mL LPS, TNFα was 421.13 pg/mL, indicating that LPS stimulation activated macrophages and enhanced TNFα secretion. On the other hand, the mangiferin 8a-treated group, norathyriol-treated group, M9-treated group, and M14-treated group, 65.13 pg/mL, 68.13 pg/mL, 58.13 pg/mL, and 54.13 pg/mL, respectively.

As described above, the mangiferin 8a-, norathyriol-, M9-, and M14-treated groups significantly suppressed TNFα production in LPS stimulation.

### < Example 8 > Suppression of IL-6 production in macrophages

The inhibitory effect of compounds on IL-6 production involved in cytokine storm was examined using mouse macrophages.

Mouse macrophages were pre-cultured with RPMI1640 medium for 1 day. After incubation, mouse macrophages were treated with 10 µM mangiferin 8a, 10 µM norathyriol, 1 µM M9, and 1 µM M14 for 1 day. Then, 10 µg/mL LPS was added to mouse macrophages and cultured for 2 days. After incubation, the culture supernatant was collected and IL-6 production was determined by enzyme-linked immunosorbent assay (ELISA). This measurement was performed using a mouse IL-6 ELISA kit (R&D).

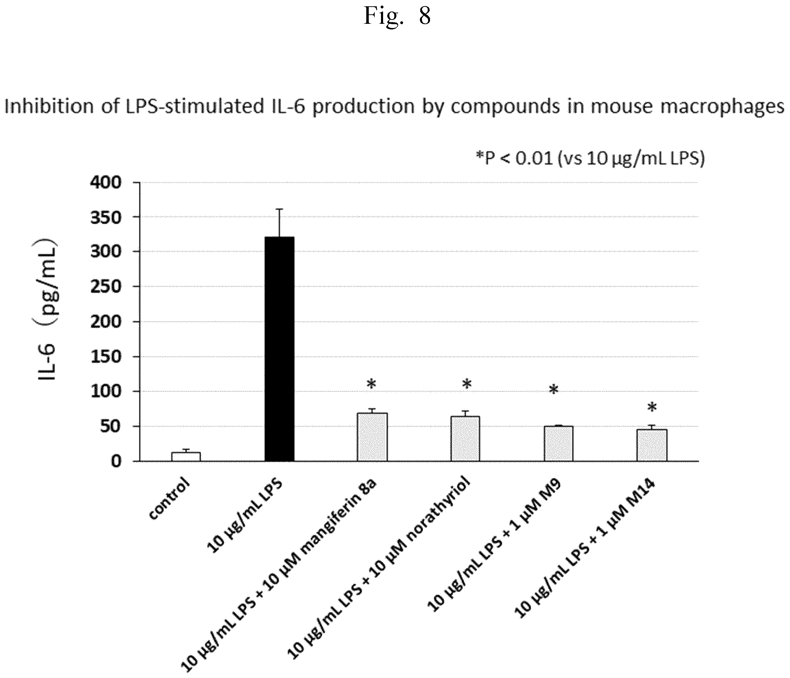

Results are shown in Figure 8. The horizontal axis represents the sample group and the vertical axis represents IL-6 production. In control, IL-6 was 13.13 pg/mL, whereas in 10 µg/mL LPS, IL-6 was 321.13 pg/mL, indicating that LPS stimulation activated B cells and enhanced IL-6 secretion. On the other hand, the mangiferin 8a-treated group, norathyriol-treated group, M9-treated group, and M14-treated group, 69.16 pg/mL, 64.13 pg/mL, 49.46 pg/mL, and 45.13 pg/mL, respectively.

As described above, the mangiferin 8a-, norathyriol-, M9-, and M14-treated groups significantly suppressed IL-6 production in LPS stimulation.

### < Example 9 > Compounds inhibit lethality in cytokine storms

Six-week-old male C57BL/6J (SHIMIZU Laboratory Supplies Co., Ltd.) were orally administered 50 mg/kg mangiferin 8a, 100 mg/kg norathyriol, 50 mg/kg M9, and 50 mg/kg M14. Five minutes later, 25 mg/kg LPS was administered intraperitoneally. The day of administration was set as day 0, and the survival rate was measured on day 2.

Those not receiving the compound and LPS are referred to as the control group, those receiving 25 mg/kg LPS are referred to as the LPS group, those receiving 50 mg/kg mangiferin 8a and 25 mg/kg LPS are referred to as the mangiferin 8a-treated group, those receiving 100 mg/kg norathyriol and 25 mg/kg LPS are referred to as the norathyriol-treated group, those receiving 50 mg/kg M9 and 25 mg/kg LPS are referred to as the M9-treated group, and those receiving 50 mg/kg M14 and 25 mg/kg LPS are referred to as the M14-treated group.

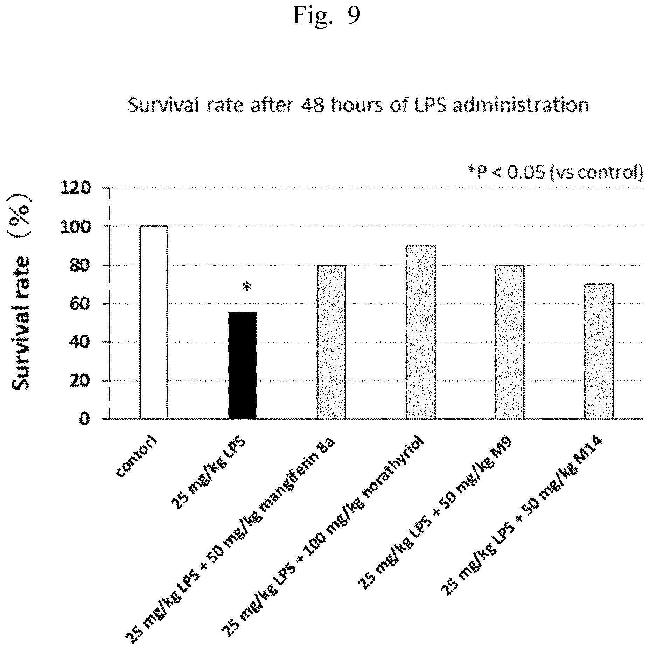

Figure 9 shows the survival rate results. Referring to Figure 9, the horizontal axis indicates compound treatment, and the vertical axis indicates survival rate (%). Those that were significantly different (P<0.05) from the control group are marked with an "*".

Figure 9 shows that compared to the control group, the LPS-treated group showed a marked decrease in survival rate, indicating that the survival rate was reduced by the cytokine storm caused by LPS administration.

Mangiferin 8a-treated group, norathyriol-treated group, M9-treated group, and M14-treated group showed significantly reduced the decrease in survival compared to the LPS group.

In other words, the mangiferin 8a-, norathyriol-, M9-, and M14-treated groups improved the lethality caused by cytokine storms.

### < Example 10 > Inhibition of TNFα production

Six-week-old male C57BL/6J (Shimizu experimental material) was orally administered 100 mg/kg norathyriol, 50 mg/kg M9, and 50 mg/kg M14. Five minutes later, 25 mg/kg LPS was administered intraperitoneally. Serum was collected from mice 2 hours after LPS administration, and the amount of TNFα production involved in the cytokine storm was measured by enzyme-linked immunosorbent assay (ELISA). This measurement was performed using a mouse TNFα ELISA kit (R&D).

Those not receiving the compound and LPS are referred to as the control group, those receiving 25 mg/kg LPS are referred to as the LPS group, those receiving 100 mg/kg norathyriol and 25 mg/kg LPS are referred to as the norathyriol-treated group, those receiving 50 mg/kg M9 and 25 mg/kg LPS are referred to as the M9-treated group, and those receiving 50 mg/kg M14 and 25 mg/kg LPS are referred to as the M14-treated group.

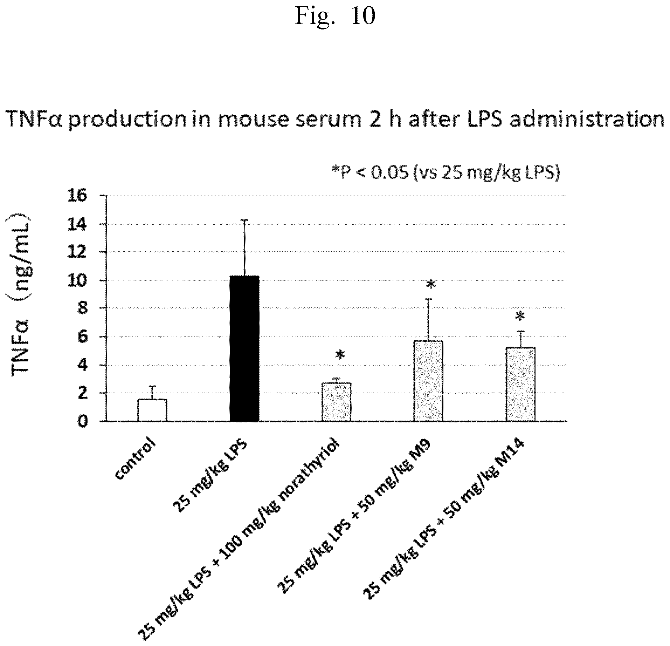

The results are shown in Figure 10. The horizontal axis represents the sample group and the vertical axis represents TNFα production. In control, TNFα was 1.55 ng/mL, whereas in 25 mg/kg LPS, TNFα was 10.28 ng/mL, indicating that it caused a cytokine storm. On the other hand, the norathyriol-treated group, M9-treated group, and M14-treated group, 2.71 ng/mL, 5.68 ng/mL, and 5.2 ng/mL, respectively.

As described above, the norathyriol-, M9-, and M14-treated groups significantly suppressed TNFα production related to cytokine storms in vivo.

### < Example 11 > Inhibition of IL-6 production

Six-week-old male C57BL/6J (Shimizu experimental material) was orally administered 100 mg/kg norathyriol, 50 mg/kg M9, and 50 mg/kg M14. Five minutes later, 25 mg/kg LPS was administered intraperitoneally. Serum was collected from mice 2 hours after LPS administration, and the amount of IL-6 production involved in the cytokine storm was measured by enzyme-linked immunosorbent assay (ELISA). This measurement was performed using a mouse IL-6 ELISA kit (R&D).

Those not receiving the compound and LPS are referred to as the control group, those receiving 25 mg/kg LPS are referred to as the LPS group, those receiving 100 mg/kg norathyriol and 25 mg/kg LPS are referred to as the norathyriol-treated group, those receiving 50 mg/kg M9 and 25 mg/kg LPS are referred to as the M9-treated group, and those receiving 50 mg/kg M14 and 25 mg/kg LPS are referred to as the M14-treated group.

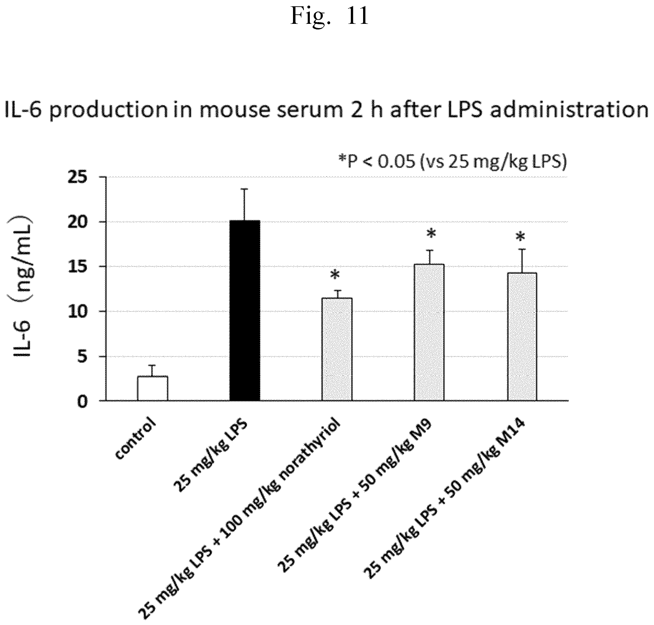

The results are shown in Figure 11. The horizontal axis represents the sample group and the vertical axis represents IL-6 production. In control, TNFα was 2.75 ng/mL, whereas in 25 mg/kg LPS, IL-6 was 20.1 ng/mL, indicating that it caused a cytokine storm. On the other hand, the norathyriol-treated group, M9-treated group, and M14-treated group, 11.37 ng/mL, 15.15 ng/mL, and 14.26 ng/mL, respectively.

As described above, the norathyriol-, M9-, and M14-treated groups significantly suppressed IL-6 production related to cytokine storms in vivo.

### [Industrial Applicability]

The cytokine storm inhibitors of the present invention can provide a therapeutic agent for cytokine storms, which are one of the causes of death, such as COVID-19, cytokine release syndrome, cytokine release syndrome due to CAR-T therapy, sepsis, systemic inflammatory response syndrome, hemophagocytic syndrome, macrophage activation syndrome, influenza, GVHD, systemic vasculitis, Kawasaki disease, Takayasu arteritis, etc. Furthermore, it can be used as a therapeutic agent for fulminant pneumonia, fulminant respiratory failure, fulminant hepatitis, fulminant myocarditis, fulminant colitis, fulminant sepsis, fulminant malaria, fulminant antiphospholipid antibody syndrome, fulminant pancreatitis, fulminant meningococcal bacteremia, fulminant type 1 diabetes, mutant fulminant collagen disease, and fulminant inflammation from disseminated intravascular coagulation syndrome other than COVID-19, cytokine release syndrome, cytokine release syndrome due to CAR-T therapy, sepsis, systemic inflammatory response syndrome, hemophagocytic syndrome, macrophage activation syndrome, influenza, GVHD, systemic vasculitis, Kawasaki disease, Takayasu arteritis, etc.

In addition, the improvement of cytokine storms caused by COVID-19, cytokine release syndrome, CAR-T therapy, sepsis, systemic inflammatory response syndrome, hemophagocytic syndrome, macrophage activation syndrome, influenza, GVHD, systemic vasculitis, Kawasaki disease, Takayasu arteritis, and others can improve the survival rate of patients. Furthermore, the cytokine storm inhibitors of the present invention can be orally administered, contributing to the improvement of the tight medical field.

## Claims

1. A pharmaceutical composition for inhibiting cytokine storms, comprising as active ingredient a compound of any of formulas (1) to (4).

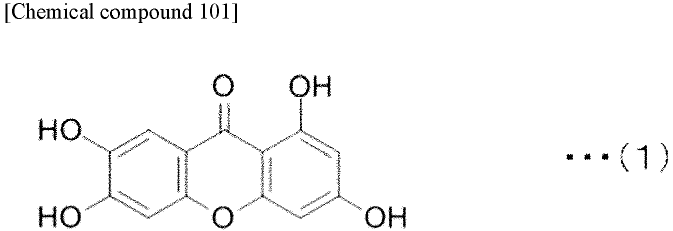

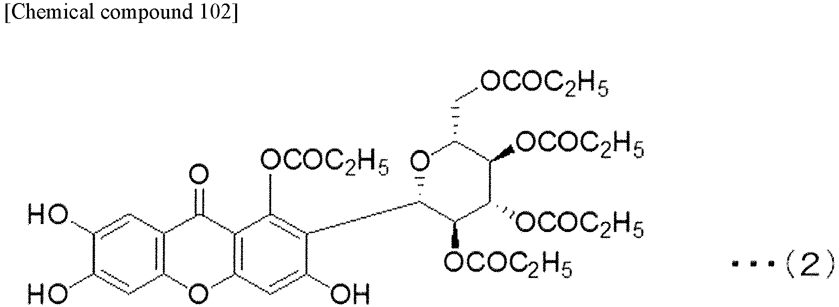

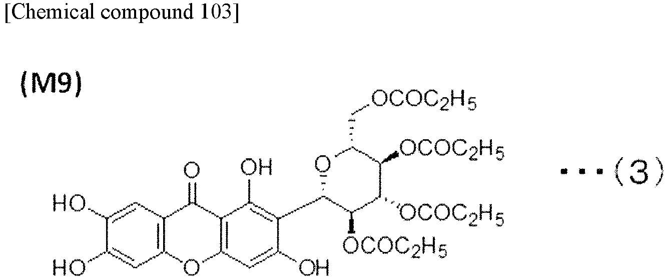

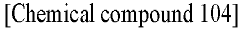

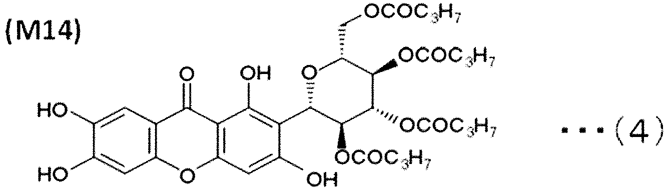

2. A pharmaceutical composition for inhibiting TNF-alpha and IL-6 production containing at least one compound of formulas (1) to (4) as described in claim 1.

3. A composition for inhibiting TNF-alpha and IL-6 production containing at least one compound of formulas (1) to (4) as described in claim 1.

4. A processed food containing at least one compound of formulas (1) to (4) as described in claim 1.

5. An agent containing at least one compound of formulas (1) to (4) as described in claim 1.
